# EUROPEAN PATENT APPLICATION

(11) **EP 2 295 041 A2**
(43) Date of publication of application: **16.03.2011**
(21) Application number: 10178130.0
(22) Date of filing: 21.09.2004
(51) Int. Cl.: A61K 9/20

(54) **HRT formulations**

(30) Priority: 29.09.2003 DK 200301408; 09.10.2003 US 509962 P; 10.12.2003 DK 200301828; 13.01.2004 US 536121 P
(62) Divisional of application: 04762858.1
(71) Applicant: Novo Nordisk Femcare AG, 8050 Zürich (CH)
(72) Inventor: Edwards, Martin William, Hants, GU34 5JF (GB); Shalmi, Michael, 2900 Hellerup (DK); Jensen, Bente Møller, 2650, Hvidovre (DK)
(74) Representative: Noergaard, Torsten

(57) **Abstract**

HRT formulations containing low amounts of estradiol and NETA are prepared.

## Description

### Field of this Invention

The present invention relates to novel pharmaceutical formulations containing surprisingly low concentrations of estradiol and norethindrone acetate (hereinafter designated NETA). These formulations are to be used for the treatment of hormone placement therapy (hereinafter designated HRT).

### Background of this lnvention

A HRT formulation designated Kliogest^{®} was marketed in the beginning of the 80'ies. Each Kliogest tablet contains, as active ingredients, 2 mg of estradiol and 1 mg of NETA. Also, at that time, it was suggested that, at least to some patients, half the dosage, i.e. a tablet containing 1 mg of estradiol and 0.5 mg of NETA, was the optimal dosage. The last mentioned product has been marketed under the name Activelle^{®}. The daily administration is one Kliogest tablet. The administration takes place for a long period of time, usually months or even years.

Examples of documents defining the general state of the art are the following patents and patent applications: US 4,826,831 A; WO 02/055086 A2; and US 2002/193358 A.

It is the object of this invention to find a HRT formulation that is extremely satisfactory to the patients.

It is the object of this invention to find a HRT formulation giving a satisfactory and convenient bleeding profile.

### Summary of this lnvention

It has now, surprisingly, been found that the object can be obtained when 0.5 mg of estradiol and 0.25 or 0.1 mg of NETA is administered daily.

In the formulations of this invention, estradiol may be present as such or it may be present as the corresponding amount of a hydrate thereof, for example, the corresponding amount of the hemihydrate (estradiol hemihydrate).

Furthermore, in the formulations of this invention, NETA may be present as such or it may be present as the corresponding amount of norethindrone (hereinafter designated NET) or the corresponding amount of a salt or ester of NET.

In a preferred embodiment, the unit dosage form is a pellet, tablet or capsule having a total weight (excluding capsule or coating) of between about 25 and 125 mg.

In some embodiments, the formulation comprises:

| | |
|---|---|
| Estradiol hemihydrate | about 0.5 mg |
| Norethisterone acetate | about 0.1 mg |
| Lactose monohydrate | about 37 mg |
| Maize starch | about 37 mg |
| Hydroxypropyl cellulose or hy-droxypropyl methyl cellulose | about 3 mg |
| Talc | about 0.8 mg |
| Magnesium stearate | about 0.4 mg |

In another aspect, the invention provides methods for treating a progestogen-responsive syndrome, which are carried out by administering to a patient in need of such treatment an effective amount for treating the syndrome of a formulation of the invention.

### Detailed Description of the lnvention

1. A pharmaceutical formulation comprising about 0.5 mg of estradiol, optionally as a hydrate thereof, and about 0.1 or about 0.25 mg of NETA or the corresponding amount of NET or a corresponding amount of an ester or a salt of NET.
2. The formulation according to embodiment 1, wherein the content of NETA is about 0.1 mg or the corresponding amount of NET or a corresponding amount of an ester or a salt of NET.
3. The formulation according to the preceding embodiment, wherein the content of NETA is about 0.1 mg.
4. The formulation according to embodiment 1, wherein the content of NETA is about 0.25 mg or the corresponding amount of NET or a corresponding amount of an ester or a salt of NET.
5. The formulation according to the preceding embodiment, wherein the content of NETA is about 0.25 mg.
6. The formulation according to any one of the preceding embodiments which is a tablet, pill, hard or soft capsule, lozenge, cachet, dispensable powder, granule, beadlet, pellet, suspension, or elixir, preferably a tablet, pill, hard or capsule.
7. The formulation according to any one of the preceding embodiments, which has a loss of mass (w/w) of not more than 15%, preferably of not more than 10 %, when tested according to European Pharmacopoeia 4^{th} edition 2002, item 2.2.32, test C.
8. The formulation according to any one of the preceding embodiments, which by the method described in European Pharmacopoeia 4^{th} edition 2002, item 2.9.1, test A, disintegrates within 2 hours, preferably 1 hour, more preferred 30 minutes, most preferred 15 minutes.
9. The formulation according to any one of the preceding embodiments, containing a cellulosic binder selected from the group consisting of methylcellulose, sodium carboxyethylellulose, (preferably Tylose™), ethylcellulose (preferably Ethocel™), hydroxypropyl methyl cellulose (hereinafter designated HPMC), and hydroxypropyl cellulose (preferably Klucel™).
10. The formulation according to any one of the preceding embodiments, wherein the content of cellulosic binder is in the range from about 2 to about 10% (w/w) relative to the total formulation.
11. The formulation according to the preceding embodiment, wherein said cellulosic binder is hydroxypropyl cellulose (preferably Klucel™).
12. The formulation according to the preceding embodiment, wherein estradiol is present as a hydrate, preferably a hemihydrate.
13. The formulation according to any one of the preceding embodiments, containing lactose in an amount in the range from about 30 to about 45 mg, optionally as lactose monohydrate.
14. The formulation according to any one of the preceding embodiments, containing maize starch in an amount in the range from about 30 to about 45 mg.
15. The formulation according to any one of the preceding embodiments, containing hydroxypropylcellulose in an amount in the range from about 2 to about 10 mg.
16. The formulation according to any one of the preceding embodiments, containing talc in an amount in the range from about 0.5 to about 2 mg.
17. The formulation according to any one of the preceding embodiments, containing magnesium stearate in an amount in the range from about 0.1 to about 1 mg.
18. The formulation according to any one of the preceding embodiments, containing hydroxypropylmethyl cellulose as film coater in an amount in the range from about 0.5 to about 3 mg.
19. The formulation according to any one of the preceding embodiments, containing glycerol triacetate in an amount in the range from about 0.05 to about 0.2 mg.
20. The formulation according to any one of the preceding embodiments, comprising an amount in the range from about 2.5 to about 4 mg of a cellulosic binder, wherein said cellulosic binder is selected from the group consisting of methylcellulose, sodium carboxymethylcellulose, ethylcellulose, hydroxypropyl methyl cellulose, and hydroxypropyl cellulose.
21. The formulation according to any one of the preceding embodiments, comprising about 40 mg of lactose monohydrate, about 40 mg of maize starch, about 3 mg of hydroxypropyl celleulose or hydroxypropyl methyl cellulose, about 0.8 mg of talc, and about 0.4 mg of magnesium stearate.
22. The formulation according to any one of the preceding embodiments, further comprising a cellulosic coating.
23. The formulation according to any one of the preceding embodiments, wherein said cellulosic coating is hydropropyl methyl cellulose.
24. The formulation according to any one of the preceding embodiments, wherein said coating is present in an amount in the range from about 0.5 to about 5% (w/w) relative to the total formulation.
25. The formulation according to the preceding embodiment, wherein said coating is present in an amount in the range from about 2 to about 4% (w/w) relative to the total formulation.
26. The formulation according to any one of the preceding embodiments, wherein said coating is present in an amount in the range from about 2.5 to about 3.5% (w/w) relative to the total formulation.
27. The formulation according to any one of the preceding embodiments, wherein the total amount of said coating is in the range from about 2 to about 3 mg per unit dosage.
28. A method for treating a progestogen-responsive syndrome, said method comprising administering to a patient in need of such treatment an effective amount for treating said syndrome of a formulation as defined in any one of the preceding product embodiments.
29. A method of providing progestogen to a subject in need of such providing, said method comprising administering to said subject a formulation as defined in any one of the preceding product embodiments.
30. Any novel feature or combination of features described herein.

The present invention relates to formulations of estradiol-containing and NETA-containing medications. Such formulations can, for example, be administered orally, vaginally, or transdermally.

The formulations of this invention can be used analogously to the use of similar products such as Kliogest and Activelle.

Examples of pharmaceutically acceptable salts, including, without limitation, organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including, without limitation, hydrochloride, sulfate, nitrate, bicarbonate, and carbonate salts.

In practicing the present invention, an estradiol-containing and NETA-containing formulation is prepared by contacting estradiola and NETA with a cellulosic binder. The binder may comprise, without limitation, one or more of methylcellulose, sodium carboxymethylcellulose (Tylose™), ethylcellulose (Ethocel™), hydroxypropyl methyl cellulose (HPMC) (Shin-Etsu Chemical Co., Ltd.), and hydroxypropyl cellulose (Klucel™). For tablet formulations, binders impart sufficient cohesion to the powders to permit normal processing such as sizing, lubrication, compression, film coating, and packaging, but still permit the tablet to disintegrate and the formulation to dissolve upon ingestion. In the present invention, the cellulosic binders also impart enhanced stability and/or recoverability to the progestogen active ingredient. Typically, the formulations of the invention comprise one or more cellulosic binders in the range of about 0.5% to about 25%, such as, e.g., about 0.75% to about 15% or about 1.5% to about 10%, of the total weight of the unit dosage form.

In one embodiment of the invention, the binder is Klucel™, and the unit dosage form is a tablet containing 0.1 or 0.25 mg NETA and 3.2 mg Klucel™ (total tablet weight = 80-85 mg).

The formulations of the invention may optionally comprise one or more diluents. Suitable diluents include, without limitation, either individually or in combination, lactose USP; lactose USP, anhydrous; lactose USP, spray dried; starch USP; directly compressible starch; mannitol USP; sorbitol; dextrose monohydrate; microcrystalline cellulose NF; dibasic calcium phosphate dihydrate NF; sucrose-based diluents; confectioner's sugar; monobasic calcium sulfate monohydrate; calcium sulfate dihydrate NF; calcium lactate trihydrate granular NF; dextrates NF (e.g., Emdex™); Celutab™; dextrose (e.g., Cerelose™); inositol; hydrolyzed cereal solids such as the Maltrons™ and Mor-Rex™; amylose; Rexcel™; calcium carbonate; glycine; bentonite; and the like. The formulations typically comprise one or more diluents in the range of about 5% to about 99%, preferably about 25% to about 90%, and more preferably about 40% to about 80%, of the total weight of the formulation.

The formulations of the invention may optionally comprise one or more disintegrants, particularly for tablet formulations. Suitable disintegrants include, without limitation, either individually or in combination, starches; sodium starch glycolate; clays (such as Veegum™HV); alginates; pregelatinized corn starches (such as National™ 1551 and National™ 1550); and gums (such as agar, guar, locust bean, Karaya™, pectin, and tragacanth). Disintegrants can be added at any suitable step during the preparation of the pharmaceutical formulation, particularly prior to granulation or during the lubrication step prior to compression. Preferably, the present pharmaceutical formulations comprise one or more disintegrants in the range of about 5% to about 99%, preferably about 25% to about 90%, and more preferably about 40% to about 80%, of the total weight of the formulation.

The formulations of the invention optionally comprise one or more lubricants and/or glidants as a carrier material. Suitable lubricants and/or glidants include, without limitation, either individually or in combination, such lubricants and/or glidants as glyceryl behenate (Compritol™ 888); metalllic stearates (e.g., magnesium, calcium and sodium stearates); stearic acid; hydrogenated vegetable oils (e.g., Sterotex™); talc; waxes; Stearowet™; boric acid; sodium benzoate and sodium acetate; sodium chloride; DL-Leucine; polyethylene glycols (e.g., Carbowax™ 4000 and Carbowax™ 6000); sodium oleate; sodium benzoate; sodium acetate; sodium lauryl sulfate; sodium stearyl fumarate (Pruv.TM.); and magnesium lauryl sulfate. The present pharmaceutical formulations comprise one or more lubricants at about 0.1 % to about 10%, preferably about 0.2% to about 8%, and more preferably about 0.25% to about 5%, of the total weight of the formulation. Magnesium stearate is a preferred lubricant used to reduce friction between the equipment and granulation during compression.

Talc is a preferred anti-adherent or glidant agent used to reduce sticking to equipment surfaces and also to reduce static in the blend. The formulations preferably comprise talc at about 0.1 % to about 10%, more preferably about 0.25% to about 5%, and still more preferably about 0.5% to about 2%, of the total weight of the formulation.

The formulations of the invention optionally comprise one or more wetting agents, particularly for tablet formulations. Suitable wetting agents include, either individually or in combination, such wetting agents as oleic acid; glyceryl monostearate; sorbitan monooleate; sorbitan monolaurate; triethanolamine oleate; polyoxyethylene sorbitan mono-oleate; polyoxyethylene sorbitan monolaurate; sodium oleate; and sodium lauryl sulfate. Wetting agents that are anionic surfactants are preferred. The formulations of the invention typically comprise one or more wetting agents present at about 0.1 % to about 15%, preferably about 0.25% to about 10%, and more preferably about 0.5% to about 5%, of the total weight of the formulation.

Other carrier materials (such as colorants, flavors and sweeteners) and modes of administration are known in the pharmaceutical art and can be used in the preparation of the formulations of the present invention.

### Dosage forms and coatings

A dosage form, as used herein, refers to a formulation that is ready for administration to a subject. In practicing the present invention, the formulation may be formulated as tablets, pills, hard or soft capsules, lozenges, cachets, dispensable powders, granules, beadlets, pellets, suspensions, elixirs, and the like. The invention also encompasses multilayered tablets wherein a given layer may represent a different drug, as well as powders, pellets and granules that are encapsulated. The powders, pellets, and granules may be coated with a suitable polymer or a conventional coating material to achieve, for example, greater stability in the gastrointestinal tract, to achieve the desired rate of release, or to achieve a product with improved stability. Moreover, the capsule comprising the powder, pellets or granules may be further coated. The tablet or the caplet may also be scored to facilitate division of dosing. Alternatively, the dosage forms of the present invention may be unit dosage forms wherein the dosage form is intended to deliver one therapeutic dose per administration.

Preferably, the formulation is formulated into a discrete dosage unit containing a predetermined amount of the active ingredients, such as tablets or capsules. Unit dosage tablets or capsules are preferred.

Coatings typically comprise one or more compounds that form a film, as well as one or more plasticizers.

Film-forming compounds include, without limitation, cellulosic compounds such as, e.g., hydroxypropyl methyl cellulose, ethylcellulose, methacrylates, cellulose acetate phthalates, polyvinyl acetate phthalates, waxes, and silicone elastomers.

Plasticizers include, without limitation, glycerin, glycerol triacetate, propylene glycol, polyethylene glycosl, triacetin, triethyl citrate, acetylated monoglycerides MACROGOL 6,000, and the like.

In one aspect, the invention provides progestogen-containing tablets coated with 1.5-5% w/w of a cellulosic film-forming compound relative to the total weight of the tablet, such as, e.g., 1.5%, 2%, 2.5%, 3%, 3.5%, 4%, 4.5, and 5% w/w.

In one embodiment, the formulation is a tablet containing 0.1 or 0.25 mg NETA and 3.2 mg Klucel™ (total tablet weight = 80-85 mg) and coated with 3% methylhydroxypropylcellulose E3 (total weight about 2.3 mg/tablet).

The formulations of the invention may be manufactured using conventional means of granulation and tabletting, including, without limitation, wet granulation and tabletting followed by film-coating or direct compression followed by film-coating.

Typically, the tablet process comprises the steps of preparing granules suitable for tableting by blending a mixture comprising the medicament, a binder, and optionally, a disintegrant and filler; adding a predetermined amount of water or granulation fluid to form a wet mass blend; sizing the wet mass blend into granules to aid drying; drying the wet granules to remove excess moisture; sizing the dried granules into granules suitable for tableting, and adding lubricant, one or more fillers, one or more dry binders, optionally a disintegrant, and other excipients necessary for tableting the granules; and a film-coating step.

Methods for preparing the formulations of the invention are well-known in the art and can be found, e.g., in Remington, 19th Edition, Vol. ll, Chapter 92, (Mack Publishing Company, Easton PA, 1995).

### Other active ingredients

In addition to progestogen, the formulations of the invention may comprise one or more additional active ingredients, including, without limitation, a second progestogen product, an androgenic agent, an androgen agonist, a progestogen agonist, an estrogen antagonist, or another hormone product.

In one embodiment, the formulation is a tablet containing 0.5 mg estradiol in addition to 0.1 or 0.25 mg N ETA.

The formulation of this invention, particularly a dry formulation such as, e.g., a tablet, can be prepared by contacting estradiol and NETA with a cellulosic binder such that the cellulosic binder comprises between about 0.5% to about 25%, such as, e.g., about 0.75% to about 15% or about 1% to about 10%, of the total weight of the formulation. Non-limiting examples of suitable cellulosic binders include methylcellulose, sodium carboxymethylcellulose (Tylose™), ethylcellulose (Ethocel™), hydroxypropyl methyl cellulose (HPMC) (Shin-Etsu Chemical Co., Ltd.), and hydroxypropyl cellulose (Klucel™). However, it will be understood that any cellulosic binder may be used that imparts enhanced stability and/or recoverability to the formulation.

In another aspect, the formulation of this isnvention, particularly a dry formulation such as, e.g., a tablet, can be prepared by coating the estrogen-containing and NETA-containing dosage form with 0.5-5% w/w of a cellulosic film-forming compound relative to the total weight of the tablet, such as, e.g., 1.5%, 2%, 2.5%, 3%, 3.5%, 4%, 4.5, and 5% w/w.

### Methods of treatment

The present invention also encompasses methods for treatment of progestogen-responsive syndromes, such as, e.g., in hormone replacement therapy. Also encompassed are other modes of progestogen administration such as, e.g., for contraception and treatment of osteoporosis. The methods are carried out by administering the formulation described above to a subject in need of a progestogen (or, a combination of a progestogen and another active ingredient, such as, e.g., an estrogen). Progestogen-responsive syndromes include, without limitation, infertility related to non-receptive uterus, premenstrual tension, ovulation, primary dysmenorrhea and endometriosis, habitual abortion, respiratory depression in the Pick-wickian syndrome, secondary amenorrhea, dysfunctional uterine bleeding, preeclampsia and toxemia of pregnancy, sexual infantilism, and post-menopausal symptoms.

The following examples are intended as non-limiting illustrations of the present invention.

### Example 1: Enhanced stability of NETA in a low-dose formulation

The purpose of this study was to compare the effect of (i) different binders and (ii) different coatings on stability of NETA is a low-dose formulation.

The following table illustrates the test formulations:

| **lngredients** | **(mg/tablet)** | **Function** | **Commercial Source** |
|---|---|---|---|
| **Active ingredients** | | | |
| Estradiol hemihydrate | 0.517 | Active substance | Schering AG, Germany / Diosynth B.V., Holland |
| Norethisterone Ace-tate | 0.100 | Active substance | Schering/Diosynth |
| **Other ingredients** | | | |
| Lactose monohydrate | 37.5 | Filler | DMV lnternational, Holland |
| Maize starch | 37.5 | Disintegrant / filler | Cerestar Scandinavia |
| **Binder** | 3.20 | Binder | |
| Talc | 0.800 | Glidant / lubricant | Luzenac, Italy |
| Magnesium stearate | 0.400 | Lubricant | Acros Chemicals |

Two different binders were tested: Polyvidon™ VA 64 (polyvinylpyrollidone, BASF, Germany); and Klucel™ EF (hydroxypropyl cellulose, Aqualon, USA).

Tabletting and coating: Tablets containing each of the three cellulosic binders were formed by fluid-bed granulation, compression on rotary press, film-coating in a coating pan using an air atomizing spray system. For film coating, either 1% or 3% Methocel E3 or E5 was used. The final tablets contained the following coat components: 1% film coat:

| | |
|---|---|
| **Film-coating: (theoretical quantity mg/tablet)** | 0.864 |
| Filmformer | 0.821 |
| Glycerol triacetate | 1.043 |

3% film coat:

| | |
|---|---|
| **Film-coating: (theoretical quantity mg/tablet)** | 2.400 |
| Filmformer | 2.280 |
| Glycerol triacetate | 0.120 |

Packaging: Following coating, each tablet batch was packed in a calendar dial pack ("Dispenser").

All patents, patent applications, and literature references referred to herein are hereby incorporated by reference in their entirety. The mentioning herein of a reference is no admission that it constitutes prior art.

Many variations of the present invention will suggest themselves to those skilled in the art in light of the above detailed description. Such obvious variations are within the full intended scope of the embodiments.

Herein the word "comprise" is to be interpreted broadly meaning "include", "contain" or "comprehend" (vide, for example, Guidelines for Examination in the European Patent Office, Part C, Chapter lll, 4.13).

## Claims

1. A pharmaceutical formulation comprising not more than about 0.5 mg of estradiol and not less than about 0.5 mg of estradiol, optionally as a hydrate thereof, and not more than about 0.1 mg of NETA and not less than about 0.1 mg of NETA or the corresponding amount of NET or a corresponding amount of an ester or a salt of NET.

2. The formulation according to the preceding claim, wherein the content of NETA is about 0.1 mg.

3. The formulation according to any one of the preceding claims which is a tablet, pill, hard or soft capsule, lozenge, cachet, dispensable powder, granule, beadlet, pellet, suspension, or elixir, preferably a tablet, pill, hard or capsule.

4. The formulation according to any one of the preceding claims, which has a loss of mass (w/w) of not more than 15%, preferably of not more than 10 %, when tested according to European Pharmacopoeia 4^{th} edition 2002, item 2.2.32, test C, and which by the method described in European Pharmacopoeia 4^{th} edition 2002, item 2.9.1, test A, disintegrates within 2 hours, preferably 1 hour, more preferred 30 minutes, most preferred 15 minutes.

5. The formulation according to any one of the preceding claims, containing a cellulosic binder selected from the group consisting of methylcellulose, sodium carboxyethylellulose, (preferably Tylose™), ethylcellulose (preferably Ethocel™), hydroxypropyl methyl cellulose (hereinafter designated HPMC), and hydroxypropyl cellulose (preferably Klucel™), and wherein the content of cellulosic binder is in the range from about 2 to about 10% (w/w) relative to the total formulation, and, preferably, said cellulosic binder is hydroxypropyl cellulose (preferably Klucel™).

6. The formulation according to any one of the preceding claims, containing lactose in an amount in the range from about 30 to about 45 mg, optionally as lactose monohydrate.

7. The formulation according to any one of the preceding claims, containing maize starch in an amount in the range from about 30 to about 45 mg.

8. The formulation according to any one of the preceding claims, containing talc in an amount in the range from about 0.5 to about 2 mg.

9. The formulation according to any one of the preceding claims, containing magnesium stearate in an amount in the range from about 0.1 to about 1 mg.

10. The formulation according to any one of the preceding claims, containing hydroxypropylmethyl cellulose as film coater in an amount in the range from about 0.5 to about 3 mg.

11. The formulation according to any one of the preceding claims, containing glycerol triacetate in an amount in the range from about 0.05 to about 0.2 mg.

12. The formulation according to any one of the preceding claims, further comprising a cellulosic coating.

13. The formulation according to any one of the preceding claims, wherein said cellulosic coating is hydropropyl methyl cellulose.

14. The formulation according to any one of the preceding claims, wherein said coating is present in an amount in the range from about 0.5 to about 5% (w/w) relative to the total formulation, preferably in an amount in the range from about 2 to about 4% (w/w) relative to the total formulation, more preferred in an amount in the range from about 2.5 to about 3.5% (w/w) relative to the total formulation.

15. A pharmaceutical formulation comprising about 0.5 mg of estradiol, optionally as a hydrate thereof, and about 0.1 or about 0.25 mg of NETA or the corresponding amount of NET or a corresponding amount of an ester or a salt of NET.
